# EUROPÄISCHE PATENTANMELDUNG

(11) **EP 4 294 053 A1**
(43) Veröffentlichungstag der Anmeldung: **20.12.2023**
(21) Anmeldenummer: 23171987.3
(22) Anmeldetag: 08.05.2023
(51) Int. Cl.: H04R 25/00, A61B 5/16

(54) **VERFAHREN SOWIE SYSTEM ZUR ANPASSUNG EINES HÖRGERÄTS AN EINEN NUTZER**

(30) Priorität: 14.06.2022 DE 102022206028
(71) Anmelder: Sivantos Pte. Ltd., Singapore 539775 (SG)
(72) Erfinder: SERMAN, Maja, 91058 Erlangen (DE); WILSON, Cecil, 91058 Erlangen (DE)
(74) Vertreter: FDST Patentanwälte

(57) **Zusammenfassung**

Um eine hohe Akzeptanz eines Nutzers (N) für ein Hörgerät (4) zu erreichen, sind erfindungsgemäß ein Verfahren sowie ein System zur Anpassung des Hörgeräts (4) vorgesehen, bei denen in einem ersten Schritt das interozeptive Bewusstsein (I) des Nutzers (N) festgestellt und in einem zweiten Schritt ein Anpassprozess zur Anpassung des Hörgeräts (4) auf Basis des festgestellten interozeptiven Bewusstseins (I) des Nutzers (N) festgelegt wird.

## Beschreibung

Die Erfindung betrifft ein Verfahren sowie ein System zur Anpassung eines Hörgeräts an einen Nutzer des Hörgeräts.

Unter Hörgerät wird vorliegend insbesondere ein Hörhilfegerät verstanden, welches zur Kompensation von Schäden eines hörgeschädigten Nutzers ausgebildet ist. Derartige Hörhilfegeräte zeichnen sich allgemein dadurch aus, dass sie an benutzerspezifischen Hörschäden angepasst sind und ein akustisches oder elektronisches Eingangssignal eines Eingangswandlers, häufig ein elektroakustischer Wandler (Mikrophon), mittels einer Signalbearbeitung aufbereiten und ein aufbereitetes Ausgangssignal an einen Ausgangswandler, häufig ebenfalls ein elektroakustischer Wandler (Lautsprecher), abgeben. Bei der Signalbearbeitung werden typischerweise nutzerspezifische, an den Hörschaden angepasste und typischerweise frequenzabhängige Verstärkungen, eine Rauschunterdrückung und/oder eine Direktionalität eingestellt. Unter Direktionalität wird allgemein verstanden, dass das Hörgerät Schallsignale, die aus einer Richtung stammen (beispielsweise aus der Position einer Schallquelle) gezielt und stärker verstärkt werden als aus anderen Raumbereichen.

Ein Hörgerät muss an den benutzerspezifischen Hörschaden angepasst werden. Bei modernen Hörhilfegeräten sind hierzu eine Vielzahl von Einstellparametern vorgesehen, zu denen jeweils geeignete Einstellwerte nutzerspezifisch ermittelt werden müssen. Dies erfolgt im Rahmen eines Anpassungsprozesses, bei dem das Hörgerät an die Hörschädigung des Nutzers angepasst wird.

Speziell bei Erstnutzern, die also zum ersten Mal ein Hörhilfegerät tragen, aber auch bei einem Umstieg auf ein neues Hörgerät, ist eine Gewöhnung des Nutzers an das Hörgerät erforderlich. Hiervon hängt auch wesentlich die Akzeptanz des Nutzers für das Hörgerät ab.

Speziell ist eine Gewöhnung an die Hörgeräte-Eigenschaften bei komplexen Hörsituationen schwierig, beispielsweise bei lauten Umgebungssituationen.

Untersuchungen haben gezeigt, dass die Akzeptanz der Nutzer stark variiert, selbst wenn die Nutzer zu einer Gruppe mit sehr ähnlichen oder auch nahezu identischen Hörschäden zählen. Dies gilt selbst dann, wenn der Anpassprozess mit Unterstützung eines Hörgeräteakustikers durchgeführt wird.

Eine besondere Hürde stellt die Akzeptanz speziell bei Erstnutzern und insbesondere bei rezeptfreien Hörgeräten dar, welche direkt ohne die Unterstützung eines Hörgeräteakustikers an die Nutzer vertrieben werden.

Ausgehend hiervon liegt der vorliegenden Erfindung die Aufgabe zugrunde, ein Verfahren sowie ein System zur Anpassung eines Hörgeräts an einen Nutzer, also an einen benutzerspezifischen Hörschaden anzugeben und zwar derart, dass eine möglichst hohe Akzeptanz für das Hörgerät beim Nutzer erzeugt wird.

Die Aufgabe wird gemäß der Erfindung gelöst durch ein Verfahren sowie ein System zur Anpassung eines Hörgeräts an einen Nutzer, speziell an den Hörschaden eines Nutzers des Hörgeräts, wobei in einem ersten Schritt zunächst ein interozeptives Bewusstsein des Nutzers festgestellt wird und im Anschluss hieran ein Anpassprozess zur Anpassung des Hörgeräts auf Basis dieses festgestellten interozeptiven Bewusstseins festgelegt wird. Das erfindungsgemäße System weist hierzu das Hörgerät sowie eine Einstellvorrichtung auf, wobei die Einstellvorrichtung dazu eingerichtet ist, das interozeptive Bewusstsein festzustellen sowie auf Basis des festgestellten interozeptiven Bewusstseins den Anpassprozess festzulegen.

Die nachfolgend im Zusammenhang mit dem Verfahren angeführten Vorteile und bevorzugten Ausführungen sind sinngemäß auch auf das System sowie umgekehrt zu übertragen.

Diese Ausgestaltung beruht auf der Erkenntnis, dass das interozeptive Bewusstsein maßgebend für die Hörwahrnehmung der hörgeschädigten Person ist.

Bisher wurde die Hypothese aufgestellt, dass kognitive Fähigkeiten in der Lage sind, die Varianz der Sprachwahrnehmung, auch als Sprachverständlichkeit bezeichnet, bei Hörgeschädigten mit ähnlichen Hörschwellen zu erklären, was sich jedoch nicht bestätigt hat.

Die Erkenntnis, dass es selbst unter erfahrenen Hörgeräteträgern Nutzer gibt, die mit bestimmten Hörgerätefunktionen nicht zurechtkommen oder sie nicht nutzen deutet darauf hin, dass einige intrinsische Wahrnehmungsprozesse durch das Hörgerät zerstört oder erschwert werden, was ein erneutes Erlernen und eine Gewöhnung erfordert, die, wenn sie nicht erfolgreich ist, zu einer mangelnden Akzeptanz und damit zur Aufgabe des Hörgeräts führt.

Das Problem der Vorhersage der Akzeptanz von Hörgeräten und der Gewöhnung an sie ist bisher nicht gelöst worden. Es war bisher unklar, wie die Eingewöhnung unterstützt und verbessert und die Wirksamkeit von Hörgeräten oder bestimmten Hörgerätefunktionen erhöht werden kann. Bisher war hierzu die Unterstützung des Nutzers durch den Hörgeräteakustiker durch Ausprobieren über Wochen, Monate und Jahre der Hörgerätenutzung erforderlich.

Untersuchungen haben nunmehr gezeigt, dass Personen mit einem hohen interozeptiven Bewusstsein eine bessere Hörwahrnehmung haben als Personen mit einem geringen interozeptiven Bewusstsein. Dies bedeutet, dass Personen trotz vergleichbarer Hörschäden allein aufgrund ihres unterschiedlichen interozeptiven Bewusstseins unterschiedliche Hörwahrnehmung zeigen. Hierunter wird verstanden, dass der Nutzer mit gutem interozeptiven Bewusstsein und der gleichen Umgebungs- und Hörbedingungen eine bessere Hörwahrnehmung aufweist, also mit der Hörsituation besser zurechtkommt, beispielsweise ein besseres Sprachverständnis aufweist als der Nutzer mit dem geringeren interozeptiven Bewusstsein.

So zeigen beispielsweise interne Untersuchungen, dass Nutzer trotz vergleichbarer Hörschäden mit einem hohen interozeptiven Bewusstsein eine beispielsweise um den Faktor 2 verbesserte Sprachwahrnehmungs-Schwelle (speech reception threshold, SRT) aufwiesen als Nutzer mit nur geringem interozeptiven Bewusstsein. Die Sprachwahrnehmungs-Schwelle ist ein Grad für die korrekte Wahrnehmung von Sprache und Worten in einer geräuschvollen Umgebung. Zur Ermittlung der SRT wird dabei derart vorgegangen, dass dem Nutzer eine bestimmte Hörsituation mit Geräusch und Sprache dargeboten wird und überprüft wird, wie hoch der Prozentsatz der korrekt verstandenen Sprache/Worte ist. Zur Festlegung eines SRT-Wertes wird beispielsweise das Verhältnis von Sprache zu Geräusch (SNR Signal to Noise Ratio) kontinuierlich verändert bis der Nutzer einen bestimmten Prozentsatz (z.B. 50%) der Sprache/Worte korrekt erfasst.

Ausgehend von dieser Erkenntnis ist erfindungsgemäß nunmehr vorgesehen, dass die Anpassung des Hörgeräts in Abhängigkeit des interozeptiven Bewusstseins vorgenommen wird. Dies bedeutet, dass in Abhängigkeit des festgestellten interozeptiven Bewusstseins unterschiedliche Anpassprozesse durchgeführt werden, dass sich also die Art der Anpassung des Hörgeräts - selbst bei identischen Hörschäden - unterscheiden.

Ein Nutzer mit einem geringen interozeptiven Bewusstsein wird typischerweise sanfter und langsamer an die Funktionalitäten und Fähigkeiten des Hörgeräts herangeführt, als ein Nutzer mit einem hohen interozeptiven Bewusstsein. Insgesamt führt dies dazu, dass der subjektive Nutzen für den jeweiligen Nutzer von diesem als besonders hoch eingestuft wird, wodurch die Akzeptanz für das Hörgerät entsprechend hoch ist.

Interozeption bezieht sich allgemein auf das (eigene) Wahrnehmen und Fühlen von Körperzuständen, insbesondere auf das Wahrnehmen von Änderungen eines Körperzustands. Unter interozeptivem Bewusstsein, auch als interozeptive Wahrnehmung bezeichnet, wird allgemein die Fähigkeit verstanden, Informationen über solche Körperzustände und speziell Änderungen von Körperzuständen nicht über die Außenwelt, sondern vielmehr aus und über eigene Körperabschnitte also quasi aus dem Inneren heraus zu erfassen.

Vorliegend wird unter interozeptives Bewusstsein insbesondere ein Grad der Übereinstimmung zwischen einem (interozeptiv) durch den Benutzer selbst (ohne externe Hilfsmittel) wahrgenommenen (physiologischen) Körperzustand (speziell eine wahrgenommene Änderung des Körperzustands) und einem tatsächlichen Körperzustand (bzw. der tatsächlichen Änderung des Körperzustands) verstanden. Speziell wird hierbei eine Eigen-Wahrnehmung des eigenen Herzschlages (Puls) betrachtet und mit dem tatsächlichen (mittels Messgerät gemessenem) Herzschlag (Puls) verglichen. Beim Herzschlag handelt es sich um einen sich periodisch ändernden Körperzustand (Zustand des Herzens).

Unter interozeptiv ermittelten Körperzustand, insbesondere Puls wird die Ermittlung des Körperzustandes (Eigenpuls) durch den Benutzer selbst ohne Hilfsmittel wie Pulsmessgerät aber auch ohne Ertasten des Pulses, speziell mittels der Finger beispielsweise am Handgelenk oder am Hals verstanden.

Daneben bestehen noch weitere Methoden, das interozeptive Bewusstsein zu bestimmen. Gemäß einer "Herzschlag-Unterscheidungs-Methode" wird die Eigenwahrnehmung des Pulses im Vergleich zu der Frequenz eines externen Signals, z.b. Tonfolge ausgewertet. Es wird also die Wahrnehmung betrachtet, inwieweit der interozeptiv erfasste Puls mit dem externen Signal übereinstimmt. Auch Hierbei ist der Grad der Übereinstimmung zwischen der Eigenwahrnehmung (der Übereinstimmung zwischen Puls und externem Signal) und der tatsächlichen Übereinstimmung zwischen Puls und externem Signal ausgewertet.

Eine weitere bekannte Variante zur Bestimmung eines interozeptiven Bewusstseins beruht auf der Auswertung eines Fragebogens zu der Eigenwahrnehmung von einem größeren Spektrum von Körperzustanden, insbesondere deren Änderungen. Speziell ist der Fragebogen zur Körperwahrnehmung (Body Perception Questionaire) von Stephen. W. Porges ein Standard.

Die Wahrnehmung von Zuständen und Eigenschaften des Gehörs, insbesondere eines Hörschadens, wie beispielsweise Tinnitus, Hörschwächen, insbesondere bei bestimmten Frequenzen oder Umgebungssituationen, fallen vorliegend nicht unter interozeptive Wahrnehmung.

Der Anpassprozess und insbesondere die Einstellwerte werden speziell auf Basis des Grades dieses interozeptiven Bewusstseins ausgewählt.

Gemäß einer bevorzugten Ausgestaltung ist das System selbst zum Ermitteln des interozeptiven Bewusstseins des Nutzers ausgebildet: Dies bedeutet, dass die Einstellvorrichtung, gegebenenfalls in Verbindung mit dem Hörgerät oder einem weiteren Gerät dazu eingerichtet ist, das interozeptive Bewusstsein selbst festzustellen und quasi zu messen. Durch diese Maßnahme wird daher durch das System insgesamt ein vollständiger Anpassungsprozess inklusive der Feststellung des interozeptiven Bewusstseins durchgeführt. Alternativ hierzu könnte ein anderweitig, beispielsweise vorab bereits ermitteltes interozeptives Bewusstseins erfasst und auf diese Weise festgelegt werden.

Verfahren zum Ermitteln des interozeptiven Bewusstseins sind allgemein bekannt und werden beispielsweise beschrieben in
Van Elk, Michiel, et al. "Suppression of the auditory N1-component for heartbeatrelated sounds reflects interoceptive predictive coding. " Biological psychology 99 (2014): 172-182*,* oder auch in
Palmer, Clare, Vivien Ainley, and Manos Tsakiris. "Fine Tuning Your Heart: a novel method for measuring interoceptive accuracy. " (2019*).*

Vorliegend wird bevorzugt ein Verfahren eingesetzt, welches auf modifizierte Versionen von verschiedenen bekannten Methoden basiert, wie es insbesondere in der Veröffentlichung von Palmer, Clare, Vivien Ainley, and Manos Tsakiris. "Fine Tuning Your Heart: a novel method for measuring interoceptive accuracy." beschrieben ist.

Weitere Veröffentlichungen, die sich mit dem interozeptiven Bewusstsein befassen sind beispielsweise
Meyerholz, Luisa, et al. "Contingent biofeedback outperforms other methods to enhance the accuracy of cardiac interoception: A comparison ofshort interventions.", Journal of behavior therapy and experimental psychiatry 63 (2019): 12-20*,*
Paulus, Martin P., Justin S. Feinstein, and Sahib S. Khalsa. "An active inference approach to interoceptive psychopathology. " Annual review of clinical psychology 15 (2019): 97-122*,*
Ring, Christopher, and Jasper Brener. "Heartbeat counting is unrelated to heartbeat detection: A comparison of methods to quantify interoception." Psychophysiology 55.9 (2018*).*

In zweckdienlicher Ausgestaltung werden bei der Festlegung des Anpassprozesses Einstellwerte für Einstellparameter des Hörgeräts auf Basis des interozeptiven Bewusstseins des Nutzers festgelegt. Unter Einstellparameter des Hörgeräts werden allgemein Parameter verstanden, die die Signalverarbeitung des Eingangssignals des Eingangswandlers im Hörgerät beeinflussen und einstellbar sind, und zwar speziell solche Parameter, die in Abhängigkeit und auf Basis des Hörschadens des hörgeschädigten Nutzers eingestellt werden.

In bevorzugter Ausgestaltung handelt es sich bei den Einstellparametern um die eingangs erwähnte Direktionalität oder auch um die Rauschunterdrückung. Es wird daher ein Grad der Direktionalität und/oder der Rauschunterdrückung eingestellt. Ein hoher Grad an Direktionalität bedeutet eine hohe Direktionalität (hohe richtungsabhängige Verstärkung), ein hoher Grad an Rauschunterdrückung bedeutet eine starke Unterdrückung von Rauschen und Störgeräuschen. Diese Parameter sind besonders bedeutend für schwierige Hörsituationen in lauten Umgebungen mit einer Vielzahl von akustischen Quellen, aus denen eine herausgefiltert werden soll. Dies entspricht beispielsweise einer Party-Situation, bei der der hörgeschädigte Nutzer innerhalb eines Raumes, in dem sich viele Personen befinden, sich mit einer bestimmten Person unterhalten will. Eine derartige Party-Situation ist im Rahmen dieser Anmeldung und allgemein für die Akzeptanz von besonderem Interesse.

In bevorzugter Ausgestaltung wird dabei mit einem zunehmendem interozeptiven Bewusstsein der Grad der Rauschunterdrückung und/oder der Grad der Direktionalität verringert. Je höher also das festgestellte interozeptive Bewusstsein ist, desto geringer wird die Rauschunterdrückung und/oder der Direktionalität eingestellt. Dies beruht darauf, dass Nutzer mit einem hohen interozeptiven Bewusstsein bereits eine bessere Hörwahrnehmung haben als Nutzer mit einem geringen interozeptiven Bewusstsein. Dies bedeutet, dass für Nutzer mit einem hohen interozeptiven Bewusstsein im Vergleich zu Nutzern mit einem geringen interozeptiven Bewusstsein weniger Unterstützung benötigen.

Daneben werden vorzugsweise die Einstellwerte weiterer Einstellparameter in Abhängigkeit des festgestellten interozeptiven Bewusstseins festgelegt. Hierbei handelt es sich beispielsweise um die Verstärkung.

Insgesamt wird eine Signalverarbeitung im Hörgerät in Abhängigkeit des interozeptiven Bewusstseins so eingestellt, dass die Unterstützung und damit der Grad der Signalverarbeitung, durch das Hörgerät bei einem Nutzer mit hohem interozeptiven Bewusstsein geringer ist als bei einem Nutzer mit geringerem interozeptiven Bewusstsein. Dies führt daher insgesamt dazu, dass die vom Hörgerät bereitgestellte Signalverarbeitung des Eingangssignals bei Nutzern mit hohem interozeptiven Bewusstsein weniger stark ist. Hierunter wird verstanden, dass das Eingangssignal weniger stark beeinflusst und verändert wird, d.h. - im Vergleich zu einem unverarbeiteten Umgebungssignal - wird ein weniger stark verändertes und damit auch weniger stark gestörtes Umgebungssignal (Schallsignal) abgegeben. Dies führt auch dazu, dass nur eine geringere Gewöhnung des Nutzers erforderlich ist. Darüber hinaus führt die geringere Signalverarbeitung auch zu einem geringeren Energieverbrauch.

Für die individuelle Anpassung der Einstellungen eines Hörgeräts an den Nutzer ist häufig ein Anpassprozess vorgesehen, bei dem die Einstellwerte im Laufe der Zeit verändert werden und sukzessive einem gewünschten Zielwert angenähert werden, welcher den Hörschaden möglichst optimal kompensiert. Dieser Vorgang wird auch als Akklimatisierung bezeichnet. Dies beruht auf der Überlegung, dass es häufig erforderlich ist, dass die Einstellwerte zunächst zu Beginn des Anpassungsprozesses noch eine vergleichsweise moderate Änderung bei der Hörwahrnehmung hervorrufen (d.h. nur eine geringe Signalbearbeitung), da andernfalls der Nutzer überfordert ist und die Akzeptanz darunter leidet.

In bevorzugter Ausgestaltung ist nunmehr bei der Festlegung des Anpassprozesses vorgesehen, dass eine Geschwindigkeit, mit der die Einstellwerte im Laufe verändert werden, also die Geschwindigkeit der Akklimatisierung, in Abhängigkeit des interozeptiven Bewusstseins eingestellt wird. Und zwar wird bevorzugt bei einem hohen interozeptiven Bewusstsein eine größere Geschwindigkeit und damit eine schnellere Einstellung des Zielwerts als bei einem Nutzer mit einem geringeren interozeptiven Bewusstsein vorgenommen. Auch durch diese Maßnahme wird daher eine Verbesserung der Akzeptanz erreicht. Die Akklimatisierung ist daher ein Parameter des Anpassungsprozesses, welcher in Abhängigkeit des festgestellten interozeptiven Bewusstseins eingestellt wird.

Bei der Durchführung des Anpassprozesses, speziell bei der Einstellung der Einstellwerte für die Einstellparameter oder auch bei der zuvor beschriebenen Festlegung der Geschwindigkeit der Akklimatisierung, ist allgemein eine Konfiguration des Hörgeräts erforderlich. Hierzu werden vorzugsweise die erforderlichen Einstellwerte von der Einstellvorrichtung ermittelt und auch an das Hörgerät übertragen, welches dadurch insofern parametriert und eingestellt wird. Der gesamte Einstellprozess erfordert in der Regel eine Vielzahl von Eingaben oder Interaktionen mit dem Nutzer, beispielsweise auch zur Feststellung des Hörschadens, um dann in Abhängigkeit hiervon die Einstellwerte festzulegen.

Bevorzugt ist eine automatische Unterstützung des Nutzers bei diesen Anpassungen durch die Einstellvorrichtung vorgesehen. Vorzugsweise sind dabei unterschiedliche Grade einer automatischen Unterstützung vorgesehen. Die automatische Unterstützung wird vorzugsweise auf Basis des festgestellten interozeptiven Bewusstseins ausgewählt. Hierunter wird verstanden, dass je nach festgestelltem interozeptiven Bewusstsein - eine hohe oder geringe Unterstützung erfolgt. Bei einer hohen Unterstützung wird insbesondere eine Menüführung dem Nutzer zur Verfügung gestellt, die die einzelnen Schritte detaillierter und genauer darstellt und dadurch eine hohe Unterstützung bietet. Umgekehrt ist bei einer geringen Unterstützung eine hiervon verschiedene Menüführung mit beispielsweise weniger Details vorgesehen. Auch diese Ausgestaltung beruht dabei auf der Erkenntnis, dass Nutzer mit hohem interozeptiven Bewusstsein ein höheres Verständnis mitbringen und damit eine geringere Unterstützung benötigen. Auch hierdurch wird insgesamt die Akzeptanz erhöht.

In einer bevorzugten Weiterbildung ist schließlich vorgesehen, dass das interozeptive Bewusstsein trainiert wird. Insbesondere erfolgt dies vorzugsweise wiederum mit Hilfe des Systems, speziell mit Hilfe der Einstellvorrichtung. Diese Ausgestaltung beruht dabei auf der Erkenntnis, dass es grundsätzlich möglich ist, das interozeptive Bewusstsein zu verbessern. Durch die Verbesserung des interozeptiven Bewusstseins wird daher insgesamt die Fähigkeit des Nutzers zur Hörwahrnehmung verbessert und damit insbesondere auch das subjektive Erfolgsempfinden und damit die Akzeptanz des Hörgeräts. Darüber hinaus wird dadurch auch erreicht, dass nur ein geringer Grad an Signalverarbeitung erforderlich ist.

Nach dem durchgeführten Training wird dann vorzugsweise der erreichte Grad des interozeptiven Bewusstseins erneut festgestellt, insbesondere mittels des Systems ermittelt und entsprechend wird der anpasste Prozess auf Basis des (verbesserten) interozeptiven Bewusstseins festgelegt. Allgemein wird hierdurch ein möglichst hohes interozeptives Bewusstsein erreicht, welches grundsätzlich zu einer verbesserten Akzeptanz führt.

Verfahren und Methoden zum Trainieren des interozeptiven Bewusstseins sind grundsätzlich bekannt, und werden beispielsweise beschrieben bei
Meyerholz, Luisa, et al. "Contingent biofeedback outperforms other methods to enhance the accuracy of cardiac interoception: A comparison of short interventions." Journal of behavior therapy and experimental psychiatry 63 (2019): 12-20*.*

Die Ermittlung des interozeptiven Bewusstseins erfolgt vorzugsweise allgemein auf Basis einer interozeptiven Wahrnehmung eines kardiologischen Signals, speziell dem Puls, durch den Nutzer selbst. Unter interozeptive Wahrnehmung wird hierbei eine Wahrnehmung des eigenen Pulses (Herzschlages) des Nutzers ohne externe Hilfsmittel und auch ohne das direkte Ertasten des Pulses beispielsweise am Handgelenk, am Hals usw. verstanden.

Für dieses Ermitteln ist dabei insbesondere in bevorzugter Ausgestaltung die Unterstützung durch ein Gerät vorgesehen, nämlich beispielsweise durch das Hörgerät selbst oder durch ein sonstiges Gerät wie beispielsweise ein portables Gerät des Nutzers, z.B. ein Smartphone. Dieses Gerät, welches Teil des gesamten Systems zur Anpassung des Hörgeräts ist, ist dabei derart ausgebildet, dass dem Nutzer eine Aufgabe gestellt wird, nämlich insbesondere seinen Puls (ohne Hilfsmittel) zu erfassen und dass weiterhin über dieses Gerät oder über ein weiteres Gerät eine Nutzereingabe erfasst wird, als Antwort auf die Aufgabe. Auf Basis der Nutzereingabe wird dann durch das System auf das interozeptive Bewusstsein zurückgeschlossen.

In bevorzugter Weiterbildung umfasst das System weiterhin ein Messgerät zur tatsächlichen Erfassung des Pulses des Nutzers. Dies ist dabei derart eingerichtet, wird also derart angesteuert, dass zeitgleich zu der Aufgabenstellung an den Nutzer der tatsächliche Puls erfasst wird und mit der Nutzereingabe verglichen wird und aus diesem Vergleich schließlich auf das interozeptive Bewusstsein zurückgeschlossen wird. Das Messgerät zur Erfassung des Pulses ist vorzugsweise das Hörgerät selbst. Das Hörgerät ist also dazu eingerichtet, den Puls des Nutzers zu messen. Hierzu wird vorzugsweise das über den eingangsseitigen Schallwandler empfangene Signal im Hinblick auf die Herzfrequenz des Nutzers des Hörgeräts, welcher dieses aktuell auch trägt, ausgewertet.

In bevorzugter Ausgestaltung wird der Nutzer allgemein über eine Menüführung bei der Ermittlung und/oder beim Training seines interozeptiven Bewusstseins unterstützt. Hierzu ist allgemein eine App vorgesehen, welche vorzugsweise auf einem Smartphone, Tablet oder einem sonstigen insbesondere portablen Wiedergabegerät implementiert ist, über welches zugleich vorzugsweise auch die Nutzereingabe dargestellt wird. Auch die Nutzereingaben sind über dieses Gerät, beispielsweise über einen Touchscreen eingebbar.

In bevorzugter Ausgestaltung wird über dieses Gerät dem Nutzer eine akustische oder optische Pulsfrequenz dargeboten sowie ergänzend eine Einstellmöglichkeit für die dargebotene Pulsfrequenz, so dass also der Nutzer die dargebotene Pulsfrequenz ändern kann.

So wird beispielsweise bei der optischen Darstellung der Pulsfrequenz ein pulsierendes Symbol, beispielsweise ein Herz auf dem Display dargestellt und mittels einer Eingabe, beispielsweise einem auf dem Display dargestellten Schieberegler, erhält der Nutzer die Möglichkeit, die Pulsfrequenz zu verändern und möglichst an die eigene wahrgenommene Pulsfrequenz anzupassen. Die Einstellung dieser vom Nutzer wahrgenommenen Pulsfrequenz stellt die Nutzereingabe dar, die mit der tatsächlich gemessenen Pulsfrequenz verglichen wird.

Alternativ zu diesem optischen Symbol wird dem Nutzer ein pulsierendes akustisches Signal, speziell über das Hörgerät dargeboten, wobei auch hier der Nutzer die Pulsfrequenz beispielsweise durch eine Eingabehilfe, wie den beschriebenen Schieberegler über das Gerät einstellen und an die eigene, wahrgenommene Pulsfrequenz anpassen kann.

Gemäß einer dritten Ausführungsvariante ist schließlich vorgesehen, dass dem Nutzer über das Gerät vorzugsweise mehrfach, beispielsweise 3-5 mal hintereinander, eine Zeitdauer vorgegeben wird und der Nutzer jeweils die Anzahl seiner Herzschläge während dieser Zeitdauer ohne Hilfsmittel und ohne Ertasten des Pulses ermitteln soll und dem entsprechenden Zählwerk eingeben soll.

Speziell für die Ermittlung oder auch beim Training des interozeptiven Bewusstseins weist das System allgemein ein Anzeigeelement, wie beispielsweise ein Mobiltelefon, Tablet usw. sowie ein Eingabeelement auf, welches durch einen Touchscreen des Anzeigeelements gebildet sein kann.

Das zuvor beschriebene Training beruht vorzugsweise ebenfalls allgemein auf der interozeptiven Wahrnehmung von kardiologischen Signalen, speziell des eigenen Pulses des Nutzers. Bevorzugt wird - ähnlich wie bei der zuvor beschriebenen Ermittlung des interozeptiven Bewusstseins - auf Basis eines kardiologischen Signals das Bewusstsein dadurch trainiert, dass dem Nutzer ergänzend noch ein Feedback dargeboten wird, beispielsweise ein haptisches oder akustisches Feedback, welches insbesondere mit dem Hörgerät selbst erzeugt ist, sodass die Wahrnehmung im Hinblick auf den Puls trainiert und geschult wird. Dieses Feedback besteht beispielsweise darin, dass dem Nutzer bevorzugt über das Hörgerät als akustisches Signal die eigene (tatsächliche) Herzfrequenz dargeboten wird, wobei im Laufe des Trainings diese Darbietung zusehends zurückgefahren wird, sodass durch diese Maßnahme die interozeptive Wahrnehmung zusehends geschult und damit verbessert wird. Bei einer haptischen Unterstützung wird dem Nutzer beispielsweise eine Vibration dargeboten.

Die erwähnte Einstellvorrichtung des Systems, mit deren Hilfe zum einen das interozeptive Bewusstsein festgestellt wird und mit dem zum anderen auch der Anpassprozess festgelegt wird, ist vorzugsweise allgemein als ein Gerät mit einer Recheneinheit gebildet, speziell durch ein insbesondere portables Gerät speziell ein nutzereigenes Gerät wie beispielsweise das bereits erwähnte Smartphone,

Tablet usw.

In der Recheneinheit sind entweder Tabellen oder Algorithmen hinterlegt, anhand derer auf Basis der Nutzereingaben auf das interozeptive Bewusstsein zurückgeschlossen wird und/oder die Einstellwerte bzw. Geschwindigkeit für die Automatisierung ausgewählt werden. Schließlich ist diese Einstellvorrichtung allgemein mit dem Hörgerät über eine geeignete Kommunikationsschnittstelle wie beispielsweise eine Bluetooth-Schnittstelle zur wechselseitigen Kommunikation und insbesondere zur Übertragung und Einspielung des ausgewählten Anpassungsprozesses mit den ausgewählten Einstellparametern ausgebildet.

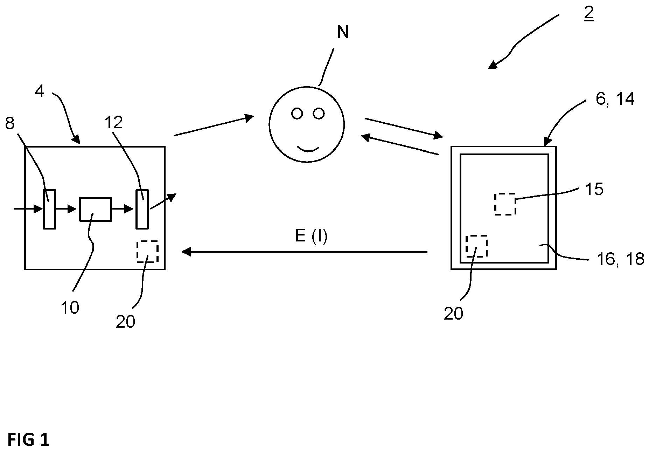

Ein Ausführungsbeispiel der Erfindung wird nachfolgend anhand der Figur näher erläutert. Diese zeigt in einer stark vereinfachten Darstellung ein System zur Anpassung eines Hörgeräts.

Das dargestellte System 2 ist zur Anpassung eines Hörgeräts 4 an den Nutzer N des Hörgeräts 4 ausgelegt. Das System 2 umfasst das Hörgerät 4 sowie im Ausführungsbeispiel ein weiteres Gerät, speziell ein mobiles Endgerät, wie ein Smartphone 6, ein Tablet oder dergleichen.

Bei dem Hörgerät 4 handelt sich um ein Hörhilfegerät, welches einen Eingangswandler 8, typischerweise ein Mikrofon, eine Signalverarbeitungseinheit 10 mit typischerweise einem Mikroprozessor sowie einen Ausgangswandler 12, typischerweise einen Lautsprecher aufweist. Ein eingehendes Signal, typischerweise ein akustisches Schallsignal, wird vom Eingangswandler 8 in ein elektrisches Eingangssignal umgewandelt, welches von der Signalverarbeitungseinheit 10 in Abhängigkeit eines nutzerspezifischen Hörschadens verarbeitet und als elektrisches Ausgangssignal an den Ausgangswandler 12 abgegeben wird, welches dieses in ein typischerweise akustisches Ausgangssignal wandelt. Grundsätzlich sind unterschiedliche Typen an Hörgeräten 4 bekannt, beispielsweise BTE, ITE oder auch CIC-Hörgeräte.

Das Hörgerät 4 muss jeweils an den Nutzer N angepasst werden. Hierzu werden insbesondere auch Einstellwerte E für verschiedene Einstellparameter, wie frequenzabhängige Verstärkungswerte zur Verstärkung des Eingangssignals, eine Rauschunterdrückung und/oder eine Direktionalität festgelegt und der Signalverarbeitungseinheit 10 mitgeteilt. Die Einstellwerte E werden im Rahmen eines Anpassungsprozesses ermittelt und eingestellt. Dies erfolgt durch eine Interaktion mit dem Nutzer N. Vorliegend werden die Einstellwerte E auch auf Basis des interozeptiven Bewusstseins I des Nutzers N ausgewählt.

Das Smartphone 6 erfüllt dabei mehrere Funktionen gleichzeitig: so übernimmt es die Funktionen einer Einstellvorrichtung 14 und nutzt hierbei seine integrierte Recheneinheit 15 aus. Weiterhin erfüllt es die Funktion eines Anzeigeelements 16 mit Hilfe seines als Touchscreen ausgebildeten Bildschirms. Darüber hinaus übernimmt es auch die Funktion einer Eingabehilfe 18, ebenfalls mit Hilfe des Touchscreens.

Der Ablauf zur Anpassung des Hörgeräts 4 ist dabei insbesondere wie folgt:
In einem ersten Schritt wird das interozeptive Bewusstsein I, auch als interozeptive Wahrnehmung bezeichnet, durch das System 2 selbst ermittelt. Hierbei stehen verschiedene Möglichkeit offen. Sämtliche Methoden beruhen dabei auf der Auswertung und Ermittlung eines kardiologischen Signals, speziell der Herzfrequenz, also des Pulses des Nutzers N. Die verschiedenen Methoden beruhen dabei darauf, dass der Nutzer N über die interozeptive Wahrnehmung seinen Puls quasi schätzt und dies über die Eingabehilfe 18 der Einstellvorrichtung 14 mitteilt. Gleichzeitig wird der tatsächliche Puls gemessen, beispielsweise über das Hörgerät 4 oder über das Smartphone 6. Der gemessene Wert wird mit dem vom Nutzer N eingegebenen Wert verglichen und hieraus wird anhand bekannter Algorithmen, wie sie aus der Literatur bekannt sind, ein Wert für das interozeptive Bewusstsein I des Nutzers N ermittelt und festgelegt.

Gemäß einer ersten Variante wird hierbei der Nutzer N dazu visuell oder akustisch aufgefordert, ein vorgegebenes Pulssignal an seinen eigenen, wahrgenommenen Puls anzupassen.

Für die visuelle Option wird dabei auf dem Anzeigeelement 16 ein pulsierendes Symbol dargestellt und der Nutzer N erhält die Aufforderung, die dargebotene Pulsfrequenz des Symbols mittels der Eingabehilfe 18, beispielsweise durch einen dargestellten Schieberegler, an seine eigene, wahrgenommene Pulsfrequenz anzupassen.

Bei einer akustischen Option wird dem Nutzer N eine Tonfolge mit einer vorgegebenen Pulsfrequenz dargeboten, beispielsweise zehn gleichmäßig voneinander beabstandete Einzeltöne, die quasi den Puls simulieren sollen. Beispielsweise kann der Nutzer N zwischen vorgegebenen, unterschiedlichen Tonfolgen mit unterschiedlichen Pulsfrequenzen auswählen, um diejenige auszuwählen, welche am besten zu dem wahrgenommenen Puls passt. Alternativ kann auch hier wiederum direkt eine Einstellung der Pulsfrequenz beispielsweise über einen Schieberegler erfolgen.

Bei einer zweiten Variante wird der Nutzer N aufgefordert, den von ihm wahrgenommenen Herzschlag über eine bestimmte Zeitdauer zu zählen. Hierzu wird von dem Anzeigeelement 16 oder auch akustisch ein Startsignal sowie ein Endsignal dem Nutzer N mitgeteilt. Der Nutzer gibt dann die von ihm wahrgenommene Anzahl an Herzschlägen ein. Dies wird vorzugsweise mehrfach wiederholt.

Das System 2 gibt weiterhin weitere ergänzende Anweisungen, beispielsweise optisch über das Anzeigeelement 16 oder auch akustisch beispielswiese über das Smartphone 6 oder auch über das Hörgerät 4, speziell wie bei der Erfassung des eigenen Pulses vorzugehen ist. Beispielsweise wird der Nutzer aufgefordert, für eine gewisse Zeit zunächst eine Ruhephase durchzuführen, sich in einem geeigneten Raum aufzuhalten, sich hinzusetzen und insbesondere, dass er für die Ermittlung seines Pulses keine Hilfsmittel heranziehen soll und auch seinen Puls nicht ertasten soll.

Wie bereits zuvor erwähnt, wird der tatsächliche Puls parallel dazu gemessen, beispielsweise über das Hörgerät 4 oder auch durch das Smartphone 6 oder durch ein sonstiges Gerät.

Aus dem Vergleich zwischen den vom Nutzer N eingegebenen Werten und den tatsächlich gemessenen Werten wird das interozeptive Bewusstsein I des Nutzers N ermittelt.

Anhand des ermittelten Werts erfolgt eine Einstufung in unterschiedliche Klassen, beispielsweise zwei bis fünf Klassen und vorzugsweise lediglich in zwei Klassen, nämlich ein niedriges sowie ein hohes interozeptives Bewusstsein I. Bei einem niedrigen Bewusstsein I hat der Nutzer eine geringere Rauschtoleranz, d.h. eine geräuschvolle Umgebung führt dazu, dass der Nutzer Sprache nur schlecht aus dieser geräuschvollen Umgebung herausfiltern kann. Dies führt dazu, dass ein solcher Nutzer einen erhöhten Bedarf an Unterstützung durch das Hörgerät 4 hat, was sich beispielsweise in einer erhöhten Rauschunterdrückung und in einer erhöhten Direktionalität zeigt. Weiterhin ist bei solchen Nutzern N typischerweise auch eine längere Akklimatisierung an das Hörgerät 4 erforderlich. D. h. die Zeitdauer, bis die Einstellwerte E auf einen gewünschten Zielwert geführt werden, ist bei derartigen Nutzern N länger als bei Nutzern N mit hohem interozeptiven Bewusstsein I.

In einem zweiten Schritt werden von der Einstellvorrichtung 14 anhand der gewählten Klasse für das interozeptive Bewusstsein I die Einstellwerte E sowie die Geschwindigkeit (Zeitdauer) für die Akklimatisierung festgelegt. Hervorzuheben ist hierbei, dass die Einstellwerte E selbst bei identischen Hörschäden von unterschiedlichen Nutzern N sich unterscheiden, wenn die Nutzer N unterschiedliche interozeptiven Wahrnehmungen aufweisen.

Speziell werden die Einstellwerte E für die Rauschunterdrückung und die Direktionalität in Abhängigkeit des interozeptiven Bewusstseins I eingestellt.

Bevorzugt ist weiterhin auch die Unterstützung, beispielsweise in der Menüführung insbesondere bei der Ermittlung des Hörschadens und der Einstellwerte E, von dem interozeptiven Bewusstsein I abhängig. Das Gleiche gilt für die Geschwindigkeit der Akklimatisierung, also die Geschwindigkeit, mit der die jeweiligen Parameter den Zielwerten angenähert werden. Diese Zeitdauer, bis die gewünschten Zielwerte der Einstellwerte E erreicht werden, kann dabei zwischen einigen Tagen und Wochen betragen.

Die von der Einstellvorrichtung 14 festgelegten Einstellwerte E sowie die Geschwindigkeit der Akklimatisierung werden über eine Kommunikationsschnittstelle 20 im Hörgerät 4 mitgeteilt und dort als Parametersatz für die Signalverarbeitungseinheit 10 hinterlegt.

### Bezugszeichenliste

- 2: System
- 4: Hörgerät
- 6: Smartphone
- 8: Eingangswandler
- 10: Signalverarbeitungseinheit
- 12: Ausgangswandler
- 14: Einstellvorrichtung
- 15: Recheneinheit
- 16: Anzeigeelement
- 18: Eingabehilfe
- 20: Kombination Schnittstelle

- N: Nutzer
- I: interozeptives Bewusstsein
- E: Einstellwert

## Patentansprüche

1. Verfahren zur Anpassung eines Hörgeräts (4) an einen Nutzer (N) des Hörgeräts (4), mit folgenden Schritten
- Feststellen des interozeptiven Bewusstseins (I) des Nutzers (N)
- Festlegung eines Anpassprozesses zur Anpassung des Hörgeräts (4) auf Basis des festgestellten interozeptiven Bewusstseins (I) des Nutzers (N).

2. Verfahren nach dem vorhergehenden Anspruch, bei dem das mit Hilfe einer Einstellvorrichtung (14) ermittelt wird, mit der auch der Anpassprozess festgelegt wird.

3. Verfahren nach einem der vorhergehenden Ansprüche, bei dem Einstellwerte (E) für Einstellparameter des Hörgeräts (4) auf Basis des interozeptiven Bewusstseins (I) des Nutzers (N) festgelegt werden.

4. Verfahren nach einem der vorhergehenden Ansprüche, bei dem in Abhängigkeit des interozeptiven Bewusstseins (I) ein Grad einer Rauschunterdrückung und/oder einer Direktionalität eingestellt wird, wobei insbesondere mit zunehmendem interozeptiven Bewusstsein (I) der Grad der Rauschunterdrückung und/oder der Grad der Direktionalität verringert wird.

5. Verfahren nach einem der vorhergehenden Ansprüche, bei dem eine Signalverarbeitungseinheit (10) des Hörgeräts (4) in Abhängigkeit des interozeptiven Bewusstseins (I) so eingestellt wird, dass die Unterstützung durch das Hörgerät (4) bei einem Nutzer (N) mit hohem interozeptiven Bewusstsein (I) geringer ist als bei einem Nutzer (N) mit geringerem interozeptiven Bewusstsein (I).

6. Verfahren nach einem der vorhergehenden Ansprüche, bei dem ein Einstellwert (E) von zumindest einem Einstellparameter des Hörgeräts (4) im Laufe des Anpassprozesses automatisch verändert wird, wobei die Geschwindigkeit der Verstellung des Einstellwerts (E) in Abhängigkeit des interozeptiven Bewusstseins (I) eingestellt wird.

7. Verfahren nach einem der vorhergehenden Ansprüche, bei dem der Nutzer (N) bei dem Anpassprozess durch eine Einstellvorrichtung (14) automatisch unterstützt wird, wobei die automatische Unterstützung des Nutzers (N) auf Basis des festgestellten interozeptiven Bewusstseins (I) ausgewählt wird.

8. Verfahren nach einem der vorhergehenden Ansprüche, bei dem das interozeptive Bewusstsein (I) des Nutzers (N) trainiert wird und vorzugsweise anschließend das interozeptive Bewusstsein (I) erneut festgestellt wird und der Anpassprozess festgelegt wird.

9. Verfahren nach einem der vorhergehenden Ansprüche, bei dem zum Ermitteln des interozeptiven Bewusstseins (I) die Erfassung eines kardiologischen Signals des Nutzers (N) durch den Nutzer (N) ohne externe Hilfsmittel vorgesehen ist, wobei der Nutzer (N) hierzu mit Hilfe einer auf einem Gerät (6) dargebotenen Menüführung unterstützt wird.

10. Verfahren nach einem der vorhergehenden Ansprüche, bei dem das Ermitteln des interozeptiven Bewusstseins (I) mit Hilfe eines Gerätes (4,6) erfolgt, nämlich mit Hilfe des Hörgeräts (4) oder mit Hilfe eines sonstigen Gerätes (6), insbesondere eines portablen Gerätes des Nutzers (N), über das dem Nutzer (N) eine Aufgabe gestellt wird, und eine Nutzereingabe des Nutzers (N) erfasst wird, und in Abhängigkeit der Nutzereingabe auf das interozeptive Bewusstsein (I) zurückgeschlossen wird, wobei die Aufgabe vorzugsweise die Erfassung des Pulses des Nutzers (N) ist, wobei gleichzeitig mit Hilfe eines Messgerätes, insbesondere des Hörgeräts (4), der tatsächliche Puls ermittelt und mit der Nutzereingabe verglichen wird.

11. Verfahren dem vorhergehenden Anspruch, bei dem dem Nutzer (N) eine akustische oder optische Pulsfrequenz sowie eine Einstellmöglichkeit für die dargebotene Pulsfrequenz angeboten wird, und wobei dem Nutzer (N) bevorzugt ein mit der Pulsfrequenz pulsierendes Symbol dargestellt wird, wobei dessen Pulsfrequenz durch den Nutzer (N) variierbar ist.

12. Verfahren nach einem der beiden vorhergehenden Ansprüche, bei dem dem Nutzer (N) insbesondere über das Hörgerät (4) eine Tonfolge mit der Pulsfrequenz präsentiert wird, wobei die Pulsfrequenz durch den Nutzer (N) variierbar ist.

13. Verfahren nach einem der Ansprüche 10 bis 12, bei dem dem Nutzer (N) vorzugsweise mehrfach eine Zeitdauer vorgegeben wird, und der Nutzer (N) zur Eingabe der von ihm während der Zeitdauer erfassten Herzschläge aufgefordert wird.

14. System zur Anpassung eines Hörgeräts (4) an einen Nutzer (N) des Hörgeräts (4), wobei das System ein Hörgerät (4) sowie eine Einstellvorrichtung (14) aufweist und die Einstellvorrichtung (14) dazu eingerichtet ist, ein interozeptives Bewusstsein (I) des Nutzers (N) festzustellen sowie auf Basis des festgestellten interozeptiven Bewusstseins (I) einen Anpassprozess zur Anpassung des Hörgeräts (4) festzulegen.

15. System nach dem vorhergehenden Anspruch, bei dem die Einstellvorrichtung (14) eine Kommunikationsschnittstelle zur Kommunikation mit dem Nutzer (N) aufweist, insbesondere ein Anzeigeelement (16), eine Eingabehilfe (18) zur Erfassung einer Eingabe des Nutzers (N) sowie eine Recheneinheit (15) aufweist, welche dazu ausgebildet ist, in Abhängigkeit von Nutzereingaben das interozeptive Bewusstsein (I) festzustellen und den Anpassprozess festzulegen, und / oder
bei dem es sich bei der Einstellvorrichtung (14) um ein insbesondere portables Gerät des Nutzers (N), wie ein Smartphone (6), Tablet oder dergleichen handelt.
